# EUROPEAN PATENT SPECIFICATION

(11) **EP 3 618 932 B1**
(45) Date of publication and mention of the grant of the patent: **01.09.2021**
(21) Application number: 18718163.1
(22) Date of filing: 20.04.2018
(51) Int. Cl.: A61Q 19/00, A61K 8/92, A61K 8/67, A61K 8/42, A61K 8/37, A61K 8/27

(54) **COSMETIC AND/OR DERMATOLOGICAL COMPOSITION, AND USES THEREOF IN THE COSMETIC FIELD AND IN THE ADJUVANT TREATMENT OF DERMATOLOGICAL PATHOLOGIES**
KOSMETISCHE UND/ODER DERMATOLOGISCHE ZUSAMMENSETZUNG UND VERWENDUNGEN DAVON IM KOSMETISCHEN BEREICH UND ZUR ADJUVANS-BEHANDLUNG VON DERMATOLOGISCHEN ERKRANKUNGEN
COMPOSITION COSMÉTIQUE ET/OU DERMATOLOGIQUE ET SES UTILISATIONS DANS LE DOMAINE COSMÉTIQUE ET DANS LE TRAITEMENT À ADJUVANT DE PATHOLOGIES DERMATOLOGIQUES

(30) Priority: 02.05.2017 IT 201700047210
(43) Date of publication of application: 11.03.2020
(73) Proprietor: Istituto Ganassini S.p.A. Di Ricerche Biochimiche, 20139 Milano (MI) (IT)
(72) Inventor: TERNO, Massimo Tarcisio, 20811 Cesano Maderno (MB) (IT)
(74) Representative: Ripamonti, Enrico
(86) International application number: PCT/EP2018/060168
(87) International publication number: WO 2018/202443

(56) References cited:
- WO-A1-2016/062522
- WO-A2-97/45098
- FR-A1- 2 993 775
- DATABASE GNPD [Online] MINTEL; September 2015 (2015-09), "After sun milk", XP002776228, Database accession no. 3408833
- DATABASE GNPD [Online] MINTEL; March 2013 (2013-03), "Tender coconut hair oil", XP002776229, Database accession no. 2023445
- DATABASE GNPD [Online] MINTEL; March 2012 (2012-03), "Shimmer lipbalm", XP002776230, Database accession no. 1751584

## Description

### TECHNICAL FIELD

The present invention refers to a cosmetic and/or dermatological composition and to its uses in the cosmetic field and as a medicament.

### BACKGROUND

Skin is one of the most important organs of the human body: it provides protection from the environment, it is responsible for our appearance and for the sense of touch. However, when the skin is dehydrated, it becomes dry and can be rough and tight, or even chapped and itchy, therefore its ability to function correctly is damaged.

Lack of hydration in the skin can be manifested in various ways, such as dryness, flaking, small cuts, redness, inflammation, constant tightness and itchiness, which can further present in various forms according to the severity and the area affected. When the skin begins to lose hydration, it becomes tight and rough. If dryness is not treated, and if the skin further loses hydration, it becomes very tight, flaky and chapped.

Dry skin is caused both by exogenous factors (like the environment and extreme climatic conditions, changes of season, ultraviolet rays) and endogenous factors (such as genetic factors, hormonal flows, age, diet). A person can be affected by several factors simultaneously, and the severity of the skin dryness will increase as the concomitant causes increase.

Knowledge of the factors that cause the skin dryness is a definite help in the prevention and treatment of this problem. The first step in the process of loss of hydration is loss of the surface lipids which form on the skin a natural barrier to prevent evaporation of the water. When this lipidic barrier is damaged, water evaporates and the vital substances that bind it are easily eliminated during washing. Because of lack of these natural moisturizing factors, the skin is not able to retain a sufficient quantity of water and becomes dry. If this condition persists, the hydration network in the deeper layers of the skin is compromised, and the flow of water towards the surface or to the upper layers of the skin is reduced, causing the skin to become very dry.

Dermatological products for the treatment of dry, reddened and chapped skin exist in the state of the art. As an example, WO 1997/45098 discloses a moisturizing and skin protective composition that consists in pure Vitamin E acetate; furthermore, a commercial product is available, that contains vitamin E acetate, panthenyl triacetate and Vitamin A and that is particularly suitable for sensitive, reddened and chapped skin, since it favours the barrier function of the epidermis with an emollient, moisturising and soothing action. Said product (marketed as Rilastil® EAP Lipogel restructuring oil) is particularly suitable since:
- it is fragrance-free;
- it has been tested for contamination levels of metals responsible for skin sensitisation (such as nickel, chromium, cobalt, mercury and palladium), with values lower than 1 ppm; but above all
- it is anhydrous (with a water activity lower than 0.6% according to UNI EN ISO 29621), and therefore free from preservatives.

Said product acts as a specific repair treatment for localized areas of the face, lips and areas of the body with sensitive, fine or irritable skin. It contains a vitamin complex, which accelerates the repair of dry, reddened and chapped skin. It favours the barrier function of the epidermis with an emollient, moisturizing and soothing action and protects the treated areas from the aggression of external agents. Due to the above-mentioned characteristics, the product responds to the requirements of tolerability, safety and effectiveness.

However, the products of the art have limits in terms of organoleptic characteristics and cosmetic pleasantness. In fact, the available compositions are difficult to distribute on the skin and remain sticky on the skin during application: for this reason, these products are only used on limited localized areas.

In order to improve the above-mentioned organoleptic and cosmetic characteristics of presently available anhydrous products, used in dermatology, ingredients like vaseline, vaseline oil and lanolin and derivatives thereof are typically added in the compositions.

Vaseline and vaseline oil consist of a mixture of hydrocarbons of mineral origin (petroleum) obtained after distillation and refinement. They are widely used as excipients of pharmaceutical and cosmetic products due to their emollient, lubricating and protective action and have the capacity to reduce trans-epidermal water loss, maintaining the correct level of hydration on the skin by means of their occlusive property. However, said products, even if used with a high degree of purity, are still of petroleum origin and can be comedogenic.

Lanolin and its derivatives, on the other hand, are of animal origin. They have emollient and moisturizing properties, a high capacity to bind with water and good adhesion on the skin; however, these compounds have various drawbacks in terms of organoleptic properties; they have also often been indicated as sensitizing agents.

Vaseline and lanolin have therefore various negative characteristics in the dermatological, chemical-physical and toxicological field.

Surprisingly, the present invention overcomes all the above-mentioned drawbacks of the known compositions, since it can be advantageously used on more extensive areas and not only on limited areas. Furthermore, it overcomes the drawbacks of vaseline and lanolin.

Indeed, the present invention provides an anhydrous product, suitable for cosmetic or dermatologic uses, that is capable of avoiding skin dehydration, safe and easy to apply even on large skin areas, besides the lack of hydrocarbons of mineral origin or lanoline and its derivatives.

Also, the product of the invention is capable of sequestering the water that is present in excess over the skin, thus avoiding the undesired growth of microbes.

FR2993775A1 discloses compositions comprising vitamin E in an amount of 1-5 wt% and D-panthenyl triacetate in an amount of 1-10wt%.

WO97/45098A2 discloses the use of pure vitamin E acetate in the treatment of various dermatological conditions.

WO2016/062522A1 discloses a composition comprising tocopheryl acetate (25 wt%) and olus oil (75 wt%).

DATABASE GNPD [Online] MINTEL (2015-09) "After sun milk" (XP002776228 - Database Acc. No. 3408833)discloses a cosmetic product comprising vitamin E, D-panthenyl triacetate and olus oil.

DATABASE GNPD [Online] MINTEL (2013-03) "Tender coconut hair oil" (XP002776229 - Database Acc. No. 2023445) discloses a hair oil cosmetic product comprising vitamin E, D-panthenyl triacetate and olus oil.

DATABASE GNPD [Online] MINTEL (2012-03) "Shimmer lipbalm" (XP002776230 - Database Acc. No. 1751584) discloses a lipbalm composition comprising bis-diglyceryl polyacyladipate-2, tocopheryl acetate and panthenyl triacetate.

### SUMMARY OF THE INVENTION

In a first embodiment, the present invention is directed to a cosmetic and/or dermatological composition comprising:
(a) Vitamin E acetate in the range 5-40% by weight of the composition (w/w);
(b) D-panthenyl triacetate in the range 2-7% (w/w);
(c) Vegetable oil Olus oil in the range 15-30% (w/w).

The components a), b) and c) listed above are overall present in the cosmetic and/or dermatological composition in a percentage lower than or equal to 100% by weight of the composition(w/w).

In a further embodiment, the present invention is directed to the composition of the present invention for use in the treatment and prevention of dermatological pathologies, correlated with skin cracking.

In another embodiment, the present invention refers to use of the composition of the present invention in the cosmetic field.

### BRIEF DESCRIPTION OF FIGURES

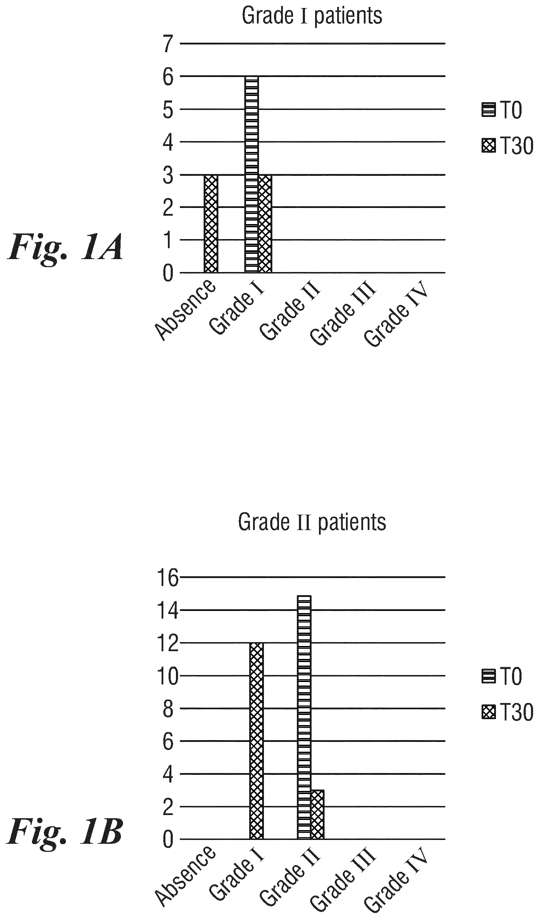

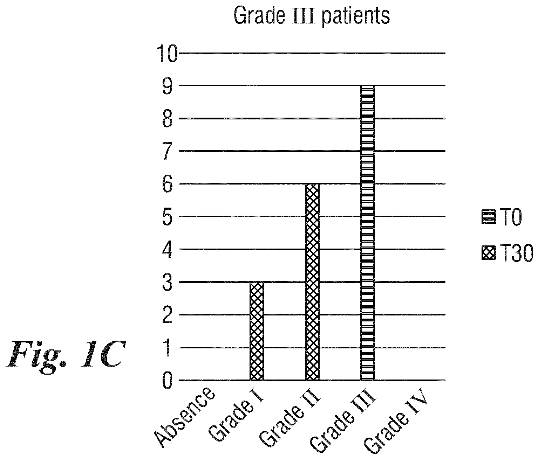

Figure 1: histogram of the results obtained by testing a composition according to a preferred embodiment of the present invention (Comp 1B) for the treatment of hemorrhoids of various severity: Grade I (Fig. 1A), Grade II (Fig. 1B) and Grade III (Fig. 1C).

### DETAILED DESCRIPTION OF THE INVENTION

The present invention refers to a composition comprising:
a) Vitamin E acetate in the range of 5-40% by weight of the composition (w/w);
b) D-panthenyl triacetate the range of 2-7% (w/w);
c) Vegetable oil Olus oil in the range 15-30% by weight of the composition (w/w).

The vegetable oil Olus Oil is a mixture consisting of triglycerides of fatty acids C16-C18 of completely vegetable origin (for example palm and palm kernel oil, coconut oil and rapeseed oil), without any additive. It is an odourless oil with a pale-yellow colour, with high stability against oxidation, a good level of dispersion and occlusiveness. It is usually found on sale under the trade name Softigen® Pura.

The components a), b) and c) listed above are overall present in the cosmetic and/or dermatological composition of the invention in an amount lower than or equal to 100% by weight of the composition (w/w), preferably in the range of 25-90% (w/w), more preferably in the range of 35-80% (w/w), even more preferably in the range of 40-70% (w/w).

Preferably, the components a), b) and c) listed above are overall present in the cosmetic and/or dermatological composition in amount approximately equal to 35% by weight of the composition (w/w), approximately equal to 40% (w/w), approximately equal to 65% (w/w), approximately equal to 85% (w/w), approximately equal to 100% (w/w).

Preferably, the composition of the present invention further comprises cosmetologically and/or dermatologically acceptable excipients, until reaching 100% by weight.

Preferably, the composition of the invention is anhydrous. In the cosmetic field the term "anhydrous" identifies compositions with a water activity lower than 0.6% (according to UNI EN ISO 29621); preferably, the composition of the invention is free from preservatives.

Preferably, the composition according to the present invention further comprises:
d) Bis-diglyceryl polyacyladipate-1 or Bis-Diglyceryl Polyacyladipate-2, or mixture of the two, in the range of 10-30% (w/w). These are substances of vegetable origin, like Olus Oil, odourless and significantly less yellow than lanolin, which show a capacity to bind water similar to lanolin, with good adhesion on the skin and an excellent toxicological profile.

Preferably, the composition according to the present invention further comprises:
e) zinc oxide in a range lower than or equal to 20% by weight of the composition (w/w), preferably in a range 4-20% (w/w). Said compound shows excellent protective properties; a composition according to a preferred embodiment of the present invention comprising zinc oxide is therefore extremely useful in preparations for the treatment of reddened skin, in dermatitis of the genital area, for example diaper dermatitis, and in the prevention of bedsores.

Preferably, the composition according to the present invention further comprises:
f) at least one compound selected from the group consisting of: jojoba oil, butyl-octyl salicylate, diethyl-hexyl-carbonate, almond oil, ethyl ester of shea butter, oleyl erucate; in which said at least one compound is present in the range of 5-30% by weight of the composition (w/w), preferably in the range of 5-15% (w/w).

Preferably, the composition according to the present invention further comprises:
g) one or more of the following compounds: triethoxy-caprylyl-silane, stearyl glycyrrhetinate, ruscogenin, neoruscogenin, acetyl tetrapeptide, laureth-9, phytic acid, Hamamelis bark extract titred in hamamelitannin, ethylic esters of vitamin F; each in a range of between 0-3% by weight of the composition (w/w).

Preferably, the composition of the present invention comprises:
a) Vitamin E acetate at about 35% (w/w);
b) D-panthenyl triacetate at about 5% (w/w);
c) Vegetable oil Olus oil at about 25% (w/w).

Preferably, the composition of the present invention comprises:
a) Vitamin E acetate at about 10% (w/w);
b) D-panthenyl triacetate at about 2.5% (w/w);
c) Vegetable oil Olus oil at about 25% (w/w).

Particularly preferred compositions (Comp 2, Comp 3, Comp 1A, Comp 1B) according to the present invention are shown below in tables 1 and 2; Comp 1 is a reference example, not according to the present invention.

**Table 1**

| **Ingredients** | **Comp 1 (w/w)** | **Comp 2 (w/w)** | **Comp 3 (w/w)** |
|---|---|---|---|
| Vitamin E Acetate | 75% | 10% | 35% |
| D-Panthenyl Triacetate | 5% | 5% | 5% |
| Olus Oil | 5-20% | ≤20 % | 24.50% |
| Zinc oxide | | ≤20 % | |
| Triethoxycaprylylsilane | | 0.25% | |
| Oleyl Erucate | | | |
| Bis-diglyceryl polyacyladipate-1 and/or Bis-diglyceryl polyacyladipate-2 | | 10% | 24.7% |
| Stearyl glycyrrhetinate | | 0.5% | 0.5% |
| neoruscogenin/ruscogenin | | | 0.30% |
| Hamamelis bark extract titred in hamamelitannin | | | |
| Acetyl tetrapeptide | | | ≥ 0.005% |
| Vitamin F (ethylic esters) | 0.30% | | |
| Laureth-9 | 0-1% | 0-1% | 0-1% |
| Phytic acid | | 0-1% | |
| Diethyl-hexyl-carbonate | | | |

**Table 2**

| **Ingredients** | **Comp 1A (w/w)** | **Comp 1B (w/w)** |
|---|---|---|
| Vitamin E Acetate | 10% | 35% |
| D-Panthenyl Triacetate | 2.50% | 5.00% |
| Olus Oil | ≤30 % | 24.80% |
| Zinc oxide | ≤20 % | |
| Triethoxycaprylylsilane | | |
| Oleyl Erucate | 6.50% | |
| Bis-diglyceryl polyacyladipate-1 and/or Bis-diglyceryl polyacyladipate-2 | 15% | 24.70% |
| Stearyl glycyrrhetinate | 0.30% | 0.50% |
| neoruscogenin/ ruscogenin | | |
| Hamamelis bark extract titred in hamamelitannin | | 3% |
| Acetyl tetrapeptide | | Min. 0.005% |
| Vitamin F (ethylic esters) | | |
| Laureth-9 | | 0-1% |
| Phytic acid | | |
| Diethyl-hexyl-carbonate | 6.50% | |
| Other excipients | Up to 100% | Up to 100% |

More preferably, in the composition Comp 1A of table 2, the amount of Olus Oil is 29% by weight (w/w) and the amount of zinc is 20% (w/w).

The compositions of the invention can be prepared using techniques known in the field, such as mixing.

For instance, a cosmetic composition can be prepared as follows: in a container, solid components are mixed under slow stirring to 80°C until a liquid and limpid mass is obtained. Powders (if present in the formulation) are added under strong stirring, keeping the temperature at 80°C; in another container, the remaining liquid components are preheated to 70°C and then added to the formulation under medium stirring; the mass is then cooled up to 30°C under slow stirring.

In a further embodiment, the present invention refers to the use of the composition of the present invention in cosmetics.

In another embodiment, the present invention refers to the composition of the present invention for use in the prevention and/or (adjuvant) treatment of dermatological pathologies.

Preferably, said dermatological pathologies are selected from the group consisting of: haemorrhoids, fissures, bedsores, dermatitis, disorders of the perianal and anorectal area, such as itching, smarting, irritation and pain, congestion of the haemorrhoidal vascular plexuses, prolapse of the mucous lining of the anorectal duct.

Therefore, the present invention refers to a composition for use in the treatment of dermatological diseases by applying the composition of the invention to one or more body areas that show or are at risk of showing skin discomfort or dermatological pathologies, such as those listed above.

The compositions according to the present invention can be applied topically (in case even internally), one or more times a day, in amounts that will vary according to the clinical conditions of the patient to be treated.

The composition of the present invention surprisingly overcomes the drawbacks of the compositions known in the state of the art and mentioned above, i.e. those with poor organoleptic and cosmetic properties; therefore, it can be advantageously used on more extensive areas of the body.

The composition of the present invention furthermore has effectiveness comparable or superior to the compositions of the state of the art in the prevention and treatment of dermatological pathologies.

### EXAMPLES

Representative non-limiting examples are illustrated below to demonstrate the advantageous properties of the present composition.

### Example 1

Preliminarily, a formulation was tested comprising Olus Oil at 6% (w/w) as the only active component, in order to evaluate the occlusive properties of the same and the ability to reduce trans epidermal water loss (TEWL). The experiments were conducted at the CDC Institute of Dermo-chemical Research, in collaboration with the University of Pavia and Bio Basic Europe Srl (protocol no. 1604C25F2).

Twenty subjects were selected, both male and female, aged between 18 and 60, in a good state of general health, with no skin pathologies or pharmacological treatments in progress, with negative anamnesis of atopy.

Patches with dimensions of 2 x 2 cm, impregnated with 2 ml of irritant gel (40% glycolic acid and 10% salicylic acid) were applied on the forearm of the subjects and kept in place for 20 minutes in order to induce a slight skin irritation. In one area of the forearm, only the patch impregnated with irritant gel was applied (control), in another area of the forearm, the composition comprising Olus Oil was also applied, before the patch.

TEWL measurements were performed with TEWAMETER®TM300, which measures the transepidermal water loss (TEWL), in terms of evaporated water on the considered skin unit: the passage from high TEWL values to normal values, in subjects whose epidermis barrier function is compromised, indicates an increase in skin hydration, whereas a TEWL increase from normal values to high values is symptomatic of damage to the barrier function of the epidermis.

Erythema measurements are performed with MEXAMETER®MX18, which is based on the principle of adsorption, using two different wavelengths: one corresponding to the absorption of haemoglobin, the other in order to avoid colorimetric influences of other pigments (e.g. bilirubin). The results obtained are shown by digital displays on a scale from 0 to 999.

The measurements were carried out:
at time T0, prior to application of the patch (basal value) and at time T0', after removal of the patch.

For the areas in which the composition comprising Olus Oil was applied, the measurement at time T0 was performed prior to application of the composition.

At time T0' an increase in the skin reddening was highlighted in 25% of the treated volunteers in the area treated with the composition of the present example (not statistically significant) and in 100% of the volunteers in the non-treated control area, where only irritant solution was applied (statistically significant).

In both the treated and non-treated areas, the irritant patch causes a significant increase in erythema, but in the area treated with the composition comprising Olus Oil, the mean erythema value increases by only 15%, whereas in the non-treated area the mean erythema increase is 101% (table 3) .

**Table 3**

| **Detection time** | **Mean erythema index treated area** | **Standard deviation** | **Mean erythema index non-treated area** | **Standard deviation** |
|---|---|---|---|---|
| **T0** | 100.35 | 10.1788 | 100.5 | 9.816742 |
| **T0'** | 115 | 12.79391 | 201.6 | 16.47774 |

In both the treated and non-treated areas, the irritant patch causes an increase in TEWL, but in the area treated with the composition comprising Olus Oil, TEWL increases on average by only 15%, whereas in the non-treated area the increase is of 107% (table 4).

**Table 4**

| **Detection times** | **Mean TEWL value treated area** | **Standard deviation** | **Mean TEWL value non-treated area** | **Standard deviation** |
|---|---|---|---|---|
| **T0** | 10.12 | 1.320526 | 9.91 | 1.064202 |
| **T0'** | 11.6 | 1.50438 | 20.53 | 2.848287 |

In view of the above, compositions according to preferred embodiments of the present invention were clinically tested to ascertain:
- adequacy of the organoleptic characteristics (viscosity, odour, adhesion);
- tolerability and effectiveness *vis-à-vis* Irritant Contact Dermatitis (ICD) and Irritant Diaper Dermatitis (IDD);
- tolerability and effectiveness for anorectal use, particularly for the prevention and adjuvant treatment of haemorrhoids and symptoms associated with disorders of the perianal and anorectal area.

### Example 2

A preliminary clinical evaluation was performed on 20 children, aged 0-8, affected by ICD and IDD, in the inflammatory phase. The composition Comp 1A of table 2, according to a preferred embodiment of the present invention, was administered topically twice a day in the case of ICD and at every diaper change in the case of IDD. During the checkup, after one month of treatment, the tolerability and clinical efficacy have been evaluated, by clinical-morphological parameters (dryness-roughness, flaking, infiltration) and subjective parameters (pruritus or burning), both before (T0) and after (T30) the daily application of the cosmetic product in the study for 1 month.

After 30 days of use there was a statistically significant reduction in the severity of the lesions and erythema, as assessed by the investigator on a scale of increasing severity from 0 to 3 (0 = absent, 1= mild, 2= moderate, 3= intense, table 5).

**Table 5**

| **Parameter** | **T0** | **T30** | **Difference (Δ)** | **p-value** | **Δ%** |
|---|---|---|---|---|---|
| **Severity of skin lesions** | 2,55± 0,14 | 0,45± 0,11 | -2,10±0,14 | P <0,0001 | -82,35% |
| **Erythema** | 2,45± 0,14 | 0,45± 0,11 | -2,00±0,16 | p<0,0001 | -81,63% |

The degree of pain perception, assessed by the mothers on the same scale as above, has decreased considerably (table 6) .

**Table 6**

| | | | | | |
|---|---|---|---|---|---|
| **Parameter** | **T0** | **T30** | **Difference (Δ)** | **p-value** | **Δ%** |
| **Pain perception** | 2,45± 0,14 | 0,30±0,11 | -2,15±0,15 | p<0,0001 | -87,75% |

No significant differences were observed between the evaluation of skin lesions by the investigator and by the mother, which was judged to be good/excellent in all cases (table 7, evaluation scores: 0= insufficient, 1= sufficient, 2= fair, 3= good, 4= excellent).

**Table 7**

| **Parameter** | **Mother** | **Investigator** | **Difference (Δ)** | **p-value** |
|---|---|---|---|---|
| **Lesions amelioration** | 3,55±0,11 | 3,45±0,11 | -0,10±0,07 | p>0,05 |

The tolerability was excellent in all cases, according to the mothers; the cosmetic characteristics been evaluated according to the rating reported in the headings of the following table 8. The data reported in table 8 represent the number of mothers that expressed each evaluation (in brackets the data are reported as percentage among all the subjects tested).

**Table 8**

| **Parameter** | **Excellent** | **Good** | **Fair** | **Sufficient** |
|---|---|---|---|---|
| **Spreading** | 8 (40%) | 9 (45%) | 3 (15%) | 0 (0%) |
| **Absorption** | 7 (35%) | 11 (55%) | 2 (10%) | 0 (0%) |
| **Perfuming** | 6 (30%) | 11 (55%) | 3 (15%) | 0 (0%) |

In summary the following results have been obtained with the composition according to a preferred embodiment of the invention:
- degree of severity of the skin lesions (doctor's evaluation): at time zero (T0) intense; after 30 days (T30) absent/slight;
- degree of reddening (doctor's evaluation): at T0 intense; at T30 absent/slight;
- pain perception (parent's evaluation): at T0 intense; at T30 absent/slight;
- side effects: no indication;
- overall evaluation of effectiveness by the doctor: good/excellent;
- overall evaluation of effectiveness by the parent: good;
- overall assessment of cosmetic pleasantness of the product by the parent: good.

### Example 3

An evaluation of the composition of the invention was carried out by proctologists who used the product for rectal exploration during examinations; in particular the composition Comp 1B of table 2, according to a preferred embodiment of the present invention, was tested.

The product was evaluated positively in relation to its consistency and practical usability since it has a lubricating action and flows easily. Furthermore, the patients treated with eight units of composition, for 8 days of treatment, did not complain of any discomfort during use of the product, encountering a reduction in itching and smarting, without the appearance of side effects. Two subjects with anal fissures reported reduction of the pain. The global effectiveness was assessed as good by the doctor and fair by the patient.

### Example 4

In order to verify the chemical-physical stability of compositions according to the present invention, stability tests were performed at ambient temperature, at 40°C (75% relative humidity, R.H.), at 4-40°C, at 50°C and at 4°C.

Tables 9-13 and 14-18 show the results obtained respectively with the preferred compositions Comp 1A and Comp 1B of table 2.

The titre of some of the main components of the product has been verified by HPLC, GC (panthenyltriacetate) and volumetric (ZnO) analyses.

**Table 9**

| **Natural ageing at ambient temperature** | | | | | |
|---|---|---|---|---|---|
| **Parameters** | **Specifications** | **T0** | **15 days** | **30 days** | **60 days** |
| **Appearance** | Shiny uniform paste | Compliant | Compliant | Compliant | Compliant |
| **Colour** | White | Compliant | Compliant | Compliant | Compliant |
| **Odour** | Charact eristic | Compliant | Compliant | Compliant | Compliant |
| **Viscosity (cP)** | 350,000 - 1,000,0 00 | 420,000 | 400,000 | 670,000 | 820,000 |
| **Vitamin E acetate** | 10% | 9.849% (98.49%) | / | / | / |
| **Zinc oxide** | 20% | 19.54% (97.72%) | / | / | / |
| **Stearyl glycyrrhetinate** | 0.3% | 0.3009% (100.3%) | / | / | / |

**Table 10**

| **Accelerated ageing at 40°C / 75% RH** | | | | | |
|---|---|---|---|---|---|
| **Parameters** | **Specifications** | **T0** | **15 days** | **30 days** | **60 days** |
| **Appearance** | Shiny uniform paste | Compliant | Compliant | Compliant | Compliant |
| **Colour** | White | Compliant | Compliant | Compliant | Compliant |
| **Odour** | Charact eristic | Compliant | Compliant | Compliant | Compliant |

**Table 11**

| **Accelerated ageing at 4-40°C** | | | | | |
|---|---|---|---|---|---|
| **Parameters** | **Specifications** | **T0** | **15 days** | **30 days** | **60 days** |
| **Appearance** | Shiny uniform paste | Compliant | Compliant | Compliant | Compliant |
| **Colour** | White | Compliant | Compliant | Compliant | Compliant |
| **Odour** | Characteristic | Compliant | Compliant | Compliant | Compliant |

**Table 12**

| **Accelerated ageing at 50°C** | | | | | |
|---|---|---|---|---|---|
| **Parameters** | **Specifications** | **T0** | **15 days** | **30 days** | **60 days** |
| **Appearance** | Shiny uniform paste | Compliant | Slightly compact, Opaque | Slightly compact, Opaque | Slightly compact, Opaque |
| **Colour** | White | Compliant | Compliant | Compliant | Compliant |
| **Odour** | Characteristic | Compliant | Compliant | Compliant | Compliant |

**Table 13**

| **Accelerated ageing at 4°C** | | | | | |
|---|---|---|---|---|---|
| **Parameters** | **Specificati ons** | **T0** | **15 days** | **30 days** | **60 days** |
| **Appearance** | Shiny uniform paste | Compliant | Compliant | Compliant | Compliant |
| **Colour** | White | Compliant | Compliant | Compliant | Compliant |
| **Odour** | Characteristic | Compliant | Compliant | Compliant | Compliant |

**Table 14**

| **Natural ageing at ambient temperature** | | | | | |
|---|---|---|---|---|---|
| **Parameters** | Specifications | T0 | 15 days | 30 days | 60 days |
| **Appearance** | Shiny Lipogel | Compliant | Compliant | Compliant | Compliant |
| **Colour** | Yellow | Compliant | Compliant | Compliant | Compliant |
| **Odour** | Characteristic | Compliant | Compliant | Compliant | Compliant |
| **Viscosity (cP)** | 45,000-90,000 | 54,000 | 88,000 | 80,000 | 80,000 |
| **Stearyl glycyrrhetinate** | 0.5 % | 0.48% (96.1%) | / | / | / |
| **Vitamin E acetate** | 35.0% | 34.67% (99.05%) | / | / | / |
| **Panthenyltriacetate** | 5.0% | 5.00% (99.9%) | / | / | / |

**Table 15**

| **Accelerated ageing at 40°C / 75% RH** | | | | | |
|---|---|---|---|---|---|
| **Parameters** | Specifications | T0 | 15 days | 30 days | 60 days |
| **Appearance** | Shiny Lipogel | Compliant | Compliant | Compliant | Compliant |
| **Colour** | Yellow | Compliant | Compliant | Compliant | Compliant |
| **Odour** | Non-perfumed | Compliant | Compliant | Compliant | Compliant |

**Table 16**

| **Accelerated ageing at 4-40°C** | | | | | |
|---|---|---|---|---|---|
| **Parameters** | Specifications | T0 | 15 days | 30 days | 60 days |
| **Appearance** | Shiny Lipogel | Compliant | Compliant | Compliant | Compliant |
| **Colour** | Yellow | Compliant | Compliant | Compliant | Compliant |
| **Odour** | Non-perfumed | Compliant | Compliant | Compliant | Compliant |

**Table 17**

| **Accelerated ageing at 50°C** | | | | | |
|---|---|---|---|---|---|
| **Parameters** | Specifications | T0 | 15 days | 30 days | 60 days |
| **Appearance** | Shiny Lipogel | Compliant | Slightly compact, Opaque | Slightly compact, Opaque | Slightly compact, Opaque |
| **Colour** | Yellow | Compliant | Compliant | Slightly darker | Slightly darker |
| **Odour** | Non-perfumed | Compliant | Compliant | Compliant | Compliant |

**Table 18**

| **Accelerated ageing at 4°C** | | | | | |
|---|---|---|---|---|---|
| **Parameters** | Specifications | T0 | 15 days | 30 days | 60 days |
| **Appearance** | Shiny Lipogel | Compliant | Compliant | Compliant | Compliant |
| **Colour** | Yellow | Compliant | Compliant | Compliant | Compliant |
| **Odour** | Non-perfumed | Compliant | Compliant | Compliant | Compliant |

After 2 months at ambient temperature, the appearance, colour, odour, and viscosity of both the compositions respond to the established specifications (tables 9, 14).

After 2 months at 40°C, the appearance, colour and odour of both the compositions respond to the specifications established (tables 10, 15).

After 2 months, the appearance, colour and odour of the product, conserved alternatively at 4°C and at 40°C for cycles of 1 week, respond to the specifications established (tables 11, 16) .

After 2 months at 50°C, the appearance, colour and odour of the product present acceptable deviations (tables 12, 17).

After 2 months at 4°C, the appearance, colour and odour of the product respond to the specifications established (tables 13, 18).

### Example 5

To verify the microbiological stability of the compositions of the present invention, a Challenge test was performed in compliance with the provisions of the European Pharmacopoeia (Ph. Eur.), current edition. The Challenge test consists in contaminating formulations to be tested with the inoculum of a test microorganisms (*Staphylococcus aureus, Pseudomonas aeruginosa, Candida albicans* and *Aspergillus brasiliensis*). Subsequently, at predetermined time intervals, a microbial load test is carried out to verify the level of contamination of the product.

In the specific case, the composition of the invention is free from preservatives, therefore the self-preserving capacity of the product is evaluated, by virtue of the absence of water (anhydrous formulation).

The microbiological stability of the formulation will be appropriate if, in the conditions of the assay, a significant reduction occurs in the number of microorganisms present in the voluntarily contaminated preparation, in the times indicated by the acceptance criteria.

The product of the invention has passed the test since it meets the required acceptance criteria expressed in the following table 19.

**Table 19**

| **Logarithmic reduction values** | | | | | |
|---|---|---|---|---|---|
| | | **2 days** | **7 days** | **14 days** | **28 days** |
| **Bacteria** | A | 2 | 3 | - | No increase |
| | B | - | - | 3 | No increase |
| **Fungi** | A | - | - | 2 | No increase |
| | B | - | - | 1 | No increase |

In fact, for both the bacteria and the fungi, the logarithmic reduction meets the criterion A established by the Ph. Eur. current edition.

Tables 20 and 21 show the results obtained respectively with the preferred compositions Comp 1A and Comp 1B of table 2.

**Table 20**

| **Test microorganisms** | **Logarithmic reduction values after:** | | | | | |
|---|---|---|---|---|---|---|
| | **0 hours** | **2 days** | **7 days** | **14 days** | **21 days** | **28 days** |
| **Gram-positive bacteria** | 1.68 | >5.28 | >5.28 | >5.28 | / | >5.28 |
| **Gram-negative bacteria** | >2.41 | >5.41 | >5.41 | >5.41 | / | >5.41 |
| **Yeasts** | >2.20 | / | >5.2 | >5.2 | / | >5.2 |
| **Moulds** | >1.45 | / | >4.45 | >4.45 | / | >4.45 |

**Table 21**

| **Test microorganisms** | **Logarithmic reduction values after:** | | | | | |
|---|---|---|---|---|---|---|
| | 0 hours | 2 days | 7 days | 14 days | 21 days | 28 days |
| **Gram-positive bacteria** | 0.26 | >5.16 | >5.16 | >5.16 | / | >5.16 |
| **Gram-negative bacteria** | >2.20 | >5.20 | >5.20 | >5.20 | / | >5.20 |
| **Yeasts** | 1.53 | / | >5.23 | >5.23 | / | >5.23 |
| **Moulds** | 1.43 | / | >4.43 | >4.43 | / | >4.43 |

### Example 6

At a gynecological clinic, the efficacy of a composition according to the invention, Comp 1B of table 2, was assessed on 30 pregnant patients with different degrees of hemorrhoids (Grade I, II and III, with increasing severity) at time 0 (T0); the tested population consists of women aged 31 ± 2 years on average, 12 of them (40%) primiparas.

After one month of treatment:
- patients with grade I hemorrhoids were reduced, with 50% of them showing no pathology after 1 month of treatment (see T30, in Figure 1A);
- patients with grade II hemorrhoids were reduced, with 80% of them evolving to grade I after 1 month of treatment (see T30, in Figure 1B);
- patients with grade III hemorrhoids were reduced, with 66.7% of them evolving to degree II and 33.3% to degree I after 1 month of treatment (see T30, in Figure 1C).

The same pattern is also noted for pain, itching, discomfort and bleeding, where in less severe cases the symptomatology improves until it almost disappears. In moderate and severe cases, the symptomatology regresses, qualitatively improving the patient's daily life.

The tolerability of treatment by patients was rated "good" in 50% of cases and "very good" in the remaining 50% of cases. The overall evaluation of the treatment, both by proctologists and patients, turned out to be very good.

The organoleptic characteristics of the composition, including the application, were also judged to be very comfortable (by 60% of patients), and the consistency non-fat. The 80% of patients considered the spreading to be good during application and the 70% considered the absorption to be fast.

### Example 7

Five different compositions, including comparative compositions available in the art and compositions according to the present invention, have been tested for their organoleptic characteristics.

In particular, the following compositions have been tested:
Comparative composition A (Comp A), consisting of pure Vitamin E acetate (it corresponds to the composition disclosed in WO 1997/45098);
Comparative composition B (Comp B), consisting of a mixture of Vitamin E acetate, in the amount of 25% by weight, and Olus Oil, in the amount of 75% by weight (it corresponds to the composition disclosed in WO 2016/062522);
Comparative composition C (Comp C), consisting of a mixture of Vitamin E acetate in the amount of 85% by weight, D-panthenyl triacetate in the amount of 11% by weight and Olus Oil in the amount of 4% by weight (it comprises the same components of the composition of the invention, but in different amounts).
Composition 1A (Comp 1A) of table 2 of the present disclosure, according to a preferred embodiment of the invention;
Composition 1B (Comp 1B) of table 2 of the present disclosure, according to a preferred embodiment of the invention.

For each composition the viscosity has been determined at 25°C with a Brookfield viscometer DV-I; both Comp A and Comp C are extremely fluid, while the other formulations (Comp B, Comp 1A and Comp 1B) have viscosities similar to creams/ointments, in line with the applications for which they are intended (see table 22).

**Table 22**

| | **Viscosity at 25°C (Rotor, G); rotation per minute, rpm)** |
|---|---|
| Comp A | 3200 cP (G=2; 10 rpm) |
| Comp B | 75000 cP (G=5; 5 rpm) |
| Comp C | 2280 cP (G=2; 10 rpm) |
| Comp 1A | 270250 cP (G=T-E95; 10 rpm) |
| Comp 1B | 54509 cP (G=T-D94; 10 rpm) |

In addition, the five compositions have been evaluated by a panel of 10 people, trained in the use of cosmetic products, who have been asked to evaluate the texture, the stickiness, the spreadability, the absorption and the overall liking of the compositions.

Texture, spreadability, absorption and liking have been scored as follows:
1 = very unpleasant; 2 = unpleasant; 3 = sufficient; 4 = good; 5 = excellent.

Stickiness has been scored as follows:
1 = excessive; 2 = average; 3 = poor; 4 = absent.

Comp A and Comp C have obtained, for all the parameters evaluated, an overall judgment between very unpleasant and unpleasant with medium and excessive stickiness.

Comp B has been evaluated with sufficient judgment for texture, spreadability and overall appreciation. Furthermore, it has been considered not very sticky. Despite this, it is reported as very unpleasant with regard to absorption.

Comp 1a and 1b have been evaluated with good judgment for all the parameters; Comp 1a is not tacky, while Comp 1b is only slightly tacky, but this is not relevant in relation to the intended use.

The mean score values are reported in table 23 for each composition.

**Tabella 23**

| | **Comp A** | **Comp B** | **Comp C** | **Comp 1A** | **Comp 1B** |
|---|---|---|---|---|---|
| **Texture** | 1,70 | 2,85 | 1,60 | 4,50 | 4,2 |
| **Spreadability** | 1,60 | 3,20 | 1,60 | 4,00 | 4,00 |
| **Absorption** | 1,30 | 1,85 | 1,35 | 4,20 | 4,30 |
| **Likeliness** | 1,30 | 2,60 | 1,60 | 4,10 | 4,00 |
| **Tackiness** | 1,30 | 3,10 | 1,55 | 4,00 | 3,20 |

Consequently, it is noted that:
- a different concentrations of the three main ingredients of the compositions (as in Comp C) with respect to the ranges envisaged by the invention, entails a loss of the good spreadability and organoleptic/cosmetic characteristics thereof;
- Comp B, that is free of D-panthenyltriacetate, is difficult to absorb (most likely in relation to presence of a too high concentrations of olus oil), thus losing the benefits of the present invention;
- Comp A (i.e. pure vitamin E acetate) has unpleasant cosmetic and organoleptic properties, in addition to being very fluid and unsuitable for applications on large surfaces.

## Claims

1. Cosmetic and/or dermatological composition comprising:
a) Vitamin E acetate in the range of 5-40% (w/w);
b) D-panthenyl triacetate in the range of 2-7%(w/w);
c) Vegetable oil Olus oil in the range of 15-30%(w/w).

2. The composition according to claim 1, further comprising:
a) Bis-diglyceryl polyacyladipate-1 or Bis-Diglyceryl Polyacyladipate-2, or mixtures thereof, in the range of 10-30% (w/w).

3. The composition according to claims 1 or 2 further comprising:
b) zinc oxide in an amount lower than or equal to 20% (w/w).

4. The composition according to any one of the preceding claims further comprising:
f) at least one compound selected from the group consisting of: jojoba oil, butyl-octyl salicylate, di-ethylhexyl carbonate, almond oil, ethyl ester of shea butter, oleyl erucate,
wherein said at least one compound is present in the range of 5-30% (w/w).

5. The composition according to any one of the preceding claims, further comprising:
g) at least one of the following compounds: triethoxycaprylylsilane, stearyl glycyrrhetinate, ruscogenin, neoruscogenin, acetyl tetrapeptide, laureth-9, phytic acid, Hamamelis bark extract titred in hamamelitannin, ethyl esters of vitamin F,
in the range of 0-3% (w/w).

6. The composition according to any one of claims 1-5, comprising:
a) Vitamin E acetate, in the range of 30-40% (w/w);
b) D-panthenyl triacetate, in the range of 3-7%(w/w);
c) Vegetable oil Olus oil, in the range of 20-30%(w/w);
d) Bis-diglyceryl polyacyladipate-1 or Bis-Diglyceryl Polyacyladipate-2, or mixtures therefore, in the range of 20-30% (w/w).

7. The composition according to any one of the preceding claims, wherein said composition is anhydrous.

8. The composition according to any one of the preceding claims, wherein said composition does not include hydrocarbons of mineral origin.

9. Non-therapeutic use of the composition according to one or more of the preceding claims in cosmetics.

10. The composition according to one or more of claims 1-8 for use in the treatment and/or prevention of dermatological pathologies.

11. The composition according to one or more of claims 1-8 for use according to claim 10, wherein the dermatological pathologies are selected from the group consisting of: haemorrhoids, fissures, bedsores, dermatitis, disorders of the perianal and anorectal area, such as itching, smarting, irritation and pain, congestion of the haemorrhoidal vascular plexuses, prolapse of the mucous lining of the anorectal duct.

12. The composition of claims 1-5 for use in the treatment of dermatitis of the anogenital area.

13. The composition of claim 6 for use in the treatment of haemorrhoids.

## Patentansprüche

1. Kosmetische und/oder dermatologische Zusammensetzung, Folgendes umfassend:
a) Vitamin-E-Acetat in der Größenordnung von 5 bis 40 Gew.-%,
b) D-Panthenyltriacetat in der Größenordnung von 2 bis 7 Gew.-%,
c) Pflanzenöl-Olus-Öl in der Größenordnung von 15 bis 30 Gew.-%.

2. Zusammensetzung nach Anspruch 1, ferner Folgendes umfassend:
a) Bis-Diglycerylpolyacyladipat-1 oder Bis-Diglycerylpolyacyladipat-2 oder Mischungen daraus in der Größenordnung von 10 bis 30 Gew.-%.

3. Zusammensetzung nach Anspruch 1 oder 2, ferner Folgendes umfassend:
b) Zinkoxid in einer Menge kleiner oder gleich 20 Gew.-%.

4. Zusammensetzung nach einem der vorhergehenden Ansprüche, ferner Folgendes umfassend:
f) mindestens eine Verbindung, die aus der Gruppe ausgewählt ist, die aus Folgendem besteht: Jojobaöl, Butyloctylsalicsylat, Dieethylhexylcarbonat, Mandelöl, Ethylester von Shea-Butter, Oleylerucat,
wobei die mindestens eine Verbindung in der Größenordnung von 5 bis 30 Gew.-% vorhanden ist.

5. Zusammensetzung nach einem der vorhergehenden Ansprüche, ferner Folgendes umfassend:
g) mindestens eine der folgenden Verbindungen: Triethoxycaprylylsilan, Stearylglycyrrhetinat, Ruscogenin, Neoruscogenin, Acetyltetrapeptid, Laureth-9, Phytinsäure, Hammamelisrindenextrakt, getitert in Hamamelitannin, Ethylester von Vitamin F,
in der Größenordnung von 0 bis 3 Gew.-%.

6. Zusammensetzung nach einem der Ansprüche 1 bis 5, Folgendes umfassend:
a) Vitamin-E-Acetat in der Größenordnung von 30 bis 40 Gew.-%,
b) D-Panthenyltriacetat in der Größenordnung von 3 bis 7 Gew.-%
c) Pflanzenöl-Olus-Öl in der Größenordnung von 20 bis 30 Gew.-%,
d) Bis-Diglycerylpolyacyladipat-1 oder Bis-Diglycerylpolyacyladipat-2 oder Mischungen daraus in der Größenordnung von 20 bis 30 Gew.-%.

7. Zusammensetzung nach einem der vorhergehenden Ansprüche, wobei die Zusammensetzung anhydrisch ist.

8. Zusammensetzung nach einem der vorhergehenden Ansprüche, wobei die Zusammensetzung keine Kohlenwasserstoffe mineralischen Ursprungs beinhaltet.

9. Nicht-therapeutische Verwendung der Zusammensetzung nach einem oder mehreren der vorhergehenden Ansprüche in Kosmetika.

10. Zusammensetzung nach einem oder mehreren der Ansprüche 1 bis 8 zur Verwendung bei der Behandlung und/oder Prävention dermatologischer Pathologien.

11. Zusammensetzung nach einem oder mehreren der Ansprüche 1 bis 8 zur Verwendung nach Anspruch 10, wobei die dermatologischen Pathologien aus der Gruppe ausgewählt sind, die aus Folgendem besteht: Hämorrhoiden, Fissuren, Druckgeschwüren, Dermatitis, Störungen des perianalen und anorektalen Bereichs, wie beispielsweise Jucken, Brennen, Reizungen und Schmerzen, Blutandrang im hämorrhoidalen vaskulären Plexus, Vorfall der Schleimhaut des Anorektalkanals.

12. Zusammensetzung nach Ansprüchen 1 bis 5 zur Verwendung bei der Behandlung von Dermatitis des anogenitalen Bereichs.

13. Zusammensetzung nach Anspruch 6 zur Behandlung von Hämorrhoiden.

## Revendications

1. Composition cosmétique et/ou dermatologique comprenant :
a) de la vitamine E acétate dans la gamme de 5-40% (p/p) ;
b) du d-panthényl triacétate dans la gamme de 2-7% (p/p) ;
c) de l'huile végétale Huile Olus dans la gamme de 15-30% (p/p).

2. Composition selon la revendication 1 comprenant en outre :
a) du bis-diglycéryl polyacyladipate-1 ou du bis-diglycéryl polyacyladipate-2, ou des mélanges de ceux-ci, dans la gamme de 10 à 30 % (p/p).

3. Composition selon la revendication 1 ou 2 comprenant en outre :
b) de l'oxyde de zinc en une quantité inférieure ou égale à 20% (p/p).

4. Composition selon l'une quelconque des revendications précédentes comprenant en outre :
f) au moins un composé choisi dans le groupe comprenant : l'huile de jojoba, le salicylate de butyle-octyle, le carbonate diéthyle-hexyle, l'huile d'amande, l'ester éthylique du beurre de karité, l'érucate d'oléyle,
dans laquelle ledit au moins un composé est présent dans la gamme de 5 à 30 % (p/p).

5. Composition selon l'une quelconque des revendications précédentes comprenant en outre :
g) au moins un des composés suivants : triéthoxycaprylylsilane, glycyrrhétinate de stéaryle, ruscogénine, néoruscogénine, tétrapeptide acétylique, laureth-9, acide phytique, extrait d'écorce d'hamamélis titré en hamamélitannine, esters éthyliques de la vitamine F, dans la gamme de 0-3% (p/p).

6. Composition selon l'une quelconque des revendications 1 à 5 comprenant :
a) de la vitamine E acétate dans la gamme de 30-40% (p/p) ;
b) du d-panthényl triacétate dans la gamme de 3-7% (p/p) ;
c) de l'huile végétale Huile Olus, dans la gamme de 20-30% (p/p);
d) du bis-diglycéryl polyacyladipate-1 ou du bis-diglycéryl polyacyladipate-2, ou des mélanges de ceux-ci, dans la gamme de 20 à 30 % (p/p).

7. Composition selon l'une quelconque des revendications précédentes, dans laquelle ladite composition est anhydre.

8. Composition selon l'une quelconque des revendications précédentes, dans laquelle ladite composition ne comprend pas des hydrocarbures d'origine minérale.

9. Utilisation non thérapeutique de la composition selon une ou plusieurs des revendications précédentes en cosmétique.

10. Composition selon une ou plusieurs des revendications 1 à 8 destinée à être utilisée dans le traitement et/ou la prévention des pathologies dermatologiques.

11. Composition selon une ou plusieurs des revendications 1 à 8 destinée à être utilisée selon la revendication 10, dans laquelle les pathologies dermatologiques sont choisies dans le groupe comprenant : les hémorroïdes, les fissures, les escarres, les dermatites, les troubles de la zone périanale et anorectale, tels que démangeaisons, picotements, irritations et douleurs, la congestion des plexus vasculaires hémorroïdaux, le prolapsus de la muqueuse du canal anorectal.

12. Composition selon la revendication 1 à 5 destinée à être utilisée dans le traitement des dermatites de la zone anogénitale.

13. Composition selon la revendication 6 destinée à être utilisée dans le traitement des hémorroïde
